# EUROPÄISCHE PATENTSCHRIFT

(11) **EP 1 678 119 B1**
(45) Veröffentlichungstag und Bekanntmachung des Hinweises auf die Patenterteilung: **05.01.2011**
(21) Anmeldenummer: 04790277.0
(22) Anmeldetag: 12.10.2004
(51) Int. Cl.: C07C 229/36, C07D 231/14

(54) **VERFAHREN ZUM HERSTELLEN VON 2-DIHALOGENACYL-3-AMINO-ACRYLS UREESTERN UND 3-DIHALOGENMETHYL-PYRAZOL-4-CARBONS UREESTERN**
METHOD FOR PRODUCING 2-DIHALOACYL-3-AMINO-ACRYLIC ACID ESTERS AND 3-DIHALOMETHYL-PYRAZOLE-4-CARBOXYLIC ACID ESTERS
PROCEDE DE PRODUCTION D'ESTERS D'ACIDE 2-DIHALOGENO-ACYL-3-AMINO-ACRYLIQUE ET D'ESTERS D'ACIDE 3-DIHALOGENO-METHYL-PYRAZOL-4-CARBOXYLIQUE

(30) Priorität: 23.10.2003 DE 10349500
(43) Veröffentlichungstag der Anmeldung: 12.07.2006
(73) Patentinhaber: Bayer CropScience AG, 40789 Monheim (DE)
(72) Erfinder: LANTZSCH, Reinhard, 42115 Wuppertal (DE); JÖRGES, Wolfgang, 51519 Odenthal (DE); PAZENOK, Sergiy, 42699 Solingen (DE)
(86) Internationale Anmeldenummer: PCT/EP2004/011376
(87) Internationale Veröffentlichungsnummer: WO 2005/042468

(56) Entgegenhaltungen:
- US-A- 5 498 624
- JAMES R BECK ET AL: "Synthesis of 1-Aryl-5-(trifluoromethyl)-1H-pyrazole-4- carboxylic Acids and Esters" JOURNAL OF HETEROCYCLIC CHEMISTRY, HETEROCORPORATION. PROVO, US, Bd. 24, Nr. 3, Mai 1987 (1987-05), Seiten 739-740, XP002126047 ISSN: 0022-152X

## Beschreibung

Die vorliegende Erfindung betrifft ein neues Verfahren zum Herstellen von neuen 2-Dihalogenacyl-3-amino-acrylsäureestern durch Umsetzung von Säurehalogeniden mit Diallcylaminoacrylsäureestern, deren Verwendung zur Herstellung von 3-Dihalogenmethyl-pyrazolen, ein Verfahren zum Herstellen von 3-Dihalogenmethyl-pyrazolen, sowie neue 3-Dihalogenmethyl-pyrazole.

Es ist bereits bekannt, dass man trihalogenacylierte Aminoacrylsäureester erhält, wenn man die entsprechenden Chloracroleine mit einem substituierten Amin umsetzt. Die als Ausgangsstoffe benötigten Chloracroleine werden dabei aus den entsprechenden Trihalogenacetoacetaten mittels Vilsmeier-Reaktion gewonnen. Dieses Verfahren hat zum einen den Nachteil, dass bei der Vilsmeier-Reaktion Phosphoroxidtrichlorid eingesetzt werden muss, und zum anderen die Gesamtausbeuten großtechnisch nicht zufriedenstellend sind (vgl. Tetrahedron Lett. 1996, 37, 8751-8754).

Weiterhin ist bekannt, dass man Trihaloacyl-aminopropenoate erhält, indem man Trihalogenacetoacetate mit Dialkylformamidacetalen umsetzt (vgl. EP-A 1 000 926). Nachteilig ist hier, dass als Nebenproduct die deacylierte Verbindung auftritt und vom gewünschten Produkt abgetrennt werden muss.

Weiterhin ist bekannt, dass man 2-Perhalogenacyl-3-amino-acrylsäure-Derivate erhalten kann, indem man 3-Aminoacrylsäureester mit Perhalogenalkylcarbonsäureanhydriden umsetzt (vgl. WO 03/051820). Dieses Verfahren eignet sich nicht zum Herstellen von dihalogenacyl-substituierten Aminoacrylsäure-Derivaten, da in Anwesenheit eines α-Wasserstoffs in Gegenwart von Triethylamin Chlorwasserstoff abgespalten wird. Die entstehenden Dihalogenketene sind sehr instabile Verbindungen (vgl. J. Org. Chem. 1968, 33, 816), welche zur Polymerisation neigen.

3-Difluormethyl-pyrazol-Derivate können erhalten werden, wenn man 2-(Difluoracetyl)-3-alkoxyacrylate mit Hydrazinen in protischen Lösungsmitteln umsetzt (vgl. US 5,498,624). Die Ausbeuten dieses Verfahrens lassen zu wünschen übrig, da zu einem hohen Prozentsatz die unerwünschten isomeren Pyrazole entstehen und bei der Isolierung des gewünschten Isomeren weitere Verluste entstehen. Die technische Anwendung eines solchen Verfahrens ist daher aus ökonomischen Gründen kaum möglich.

Bei der Ringschluss-Reaktion von Alkoxyacrylaten mit Hydrazinderivaten entsteht zu einem hohen Prozentsatz (bis 88 %) das nicht erwünschte 5-Haloalkyl-4-carbonsäure-pyrazol (vgl. J. Het. Chem. 1987, 24, 693).

Die Dihalogenmethyl-alkoxyacrylate werden aus Dihalogenacetessigester hergestellt. Dihalogenacetessigester sind technisch nicht verfügbar und erfordern aufwändige Technologie (Einsatz von Keten). Solche Verbindungen sind daher nicht in wirtschaftlicher Weise herstellbar.

Aus WO 03/051820 ist bekannt, dass man 2-Perhalogenacyl-3-amino-acrylsäure-Derivate mit Hydrazinen zu 3-Perhalogen-substituierten Pyrazolen umsetzen kann. Durch Verwendung eines nicht protischen Lösungsmittels wird die Bildung des unerwünschten Isomeren herabgesetzt, ist aber bei Anwendung auf die erfindungsgemäßen Dihalogen-Verbindungen immer noch beträchtlich (Vergleichsbeispiel 3).

Die Aufgabe der vorliegenden Erfindung bestand also in der Bereitstellung neuer, wirtschaftlicher Verfahren, durch die 2-Dihalogenacyl-3-amino-acrylsäureester mit hoher Gesamtausbeute und hoher Reinheit erhalten und mit hoher Selektivität in die gewünschten 3-Dihalogenmethyl-pyrazol-4-carbonsäureester umgesetzt werden können.

Gegenstand der vorliegenden Erfindung ist also ein Verfahren zum Herstellen von 2-Dihalogenacyl-3-amino-acrylsäureestem der Formel (I) 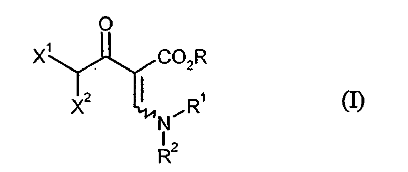 in welcher
R, R¹ und R² unabhängig voneinander für C₁-C₄-Alkyl stehen und
X¹ und X² unabhängig voneinander für Fluor, Chlor oder Brom stehen,
**dadurch gekennzeichnet, dass** man
a) Säurehalogenide der Formel (II) 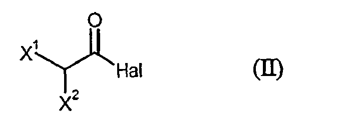 in welcher
   Hal für Fluor, Chlor oder Brom steht und
   X¹ und X² unabhängig voneinander für Fluor, Chlor oder Brom stehen,
   mit Dialkylaminoacrylsäureestern der Formel (III) 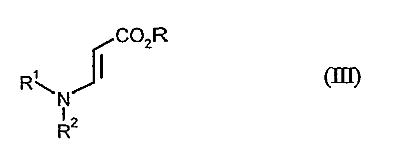 in welcher R, R¹ und R² die oben angegebenen Bedeutungen haben,
   in einem mit Wasser nicht mischbaren organischen Lösungsmittel in Gegenwart einer Base umsetzt.

Überraschenderweise lassen sich die 2-Dihalogenacyl-3-amino-acrylsäureester der Formel (I) unter den erfindungsgemäßen Bedingungen mit guten Ausbeuten in hoher Reinheit herstellen, womit das erfindungsgemäße Verfahren die oben genannten Nachteile ähnlicher bekannter Herstellungsverfahren überkommt. Insbesondere wurde überraschenderweise gefunden, dass mit Pyridin oder Pyridin-Derivaten im Gegensatz zu anderen tertiären Aminen wie z.B. Triethylamin die Dihalogenketen-Bildung unterdrückt wird und dadurch besonders hohe Ausbeuten erzielt werden.

Verwendet man Dichloracetylchlorid und Dimethylaminoacrylsäureethylester als Ausgangsstoffe und Natriumhydroxid als Base, kann das erfindungsgemäße Verfahren (a) durch das folgende Formelschema veranschaulicht werden.

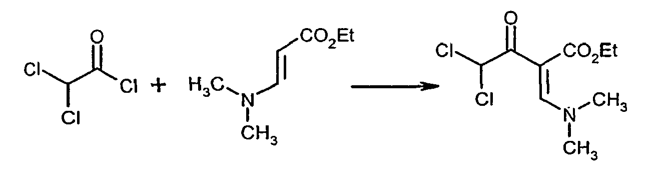

Die bei der Durchführung des erfindungsgemäßen Verfahrens (a) als Ausgangsstoffe verwendeten Säurehalogenide sind durch die Formel (II) allgemein definiert. In dieser Formel steht Hal bevorzugt für Fluor oder Chlor, besonders bevorzugt für Chlor. X steht bevorzugt für Fluor oder Chlor.

Säurehalogenide der Formel (II) sind bekannte Synthesechemikalien.

Die bei der Durchführung des erfindungsgemäßen Verfahrens (a) als Ausgangsstoffe weiterhin verwendeten Dialhylaminoacrylsäureester sind durch die Formel (III) allgemein definiert. In dieser Formel stehen R, R¹ und R² unabhängig voneinander bevorzugt für Methyl, Ethyl, n-Propyl oder iso-Propyl. R steht besonders bevorzugt für Methyl oder Ethyl, R¹ und R² stehen unabhängig voneinander besonders bevorzugt für Methyl oder Ethyl, ganz besonders bevorzugt jeweils für Methyl.

Dialkylaminoacrylsäureester der Formel (III) sind bekannte Synthesechemikalien.

Das erfindungsgemäße Verfahren (a) wird in Anwesenheit eines mit Wasser nicht mischbaren organischen Lösungsmittels durchgeführt. Hierzu gehören vorzugsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan. Besonders bevorzugt verwendet man Toluol, Xylol, Chlorbenzol, n-Hexan, Cyclohexan oder Methylcyclohexan, ganz besonders bevorzugt Toluol oder Xylol.

Das erfindungsgemäße Verfahren (a) wird in Gegenwart einer Base durchgeführt. Hierfür eignen sich besonders anorganische wässrige Basen, Pyridin oder Pyridin-Derivate. Vorzugsweise verwendbar sind Erdalkalimetall- oder Alkalimetallhydroxide, -carbonate oder -hydrogencarbonate, wie z.B. Natriumhydroxid, Kaliumhydroxid, Lithiumhydroxid, Calciumhydroxid, Natriumcarbonat, Kaliumcarbonat, Kaliumhydrogencarbonat oder Natriumhydrogencarbonat. Besonders bevorzugt verwendet man Natriumhydroxid, Kaliumhydroxid, Lithiumhydroxid, Calciumhydroxid, Natriumcarbonat oder Natriumhydrogencarbonat, ganz besonders bevorzugt Natriumhydroxid oder Kaliumhydroxid. Die anorganische Base wird vorzugsweise als wässrige Lösung in Konzentrationen zwischen 10 und 40 % eingesetzt. Weiterhin werden als Basen bevorzugt Pyridin, 2-, 3- oder 4-Methylpyridin, 2-Methyl-5-ethyl-pyridin, 2,4,6-Collidin, 2- oder 4-n-Propylpyridin, 4-Dimethylaminopyridin, Chinolin oder Chinaldin verwendet, besonders bevorzugt Pyridin, 2-Methyl-5-ethyl-pyridin, 2,4,6-Collidin, Chinolin oder Chinaldin.

Bei der Durchführung des erfindungsgemäßen Verfahrens (a) muss innerhalb eines relativ kleinen Temperaturbereichs gearbeitet werden. Im Allgemeinen arbeitet man bei Temperaturen von -20°C bis +50°C, bevorzugt bei Temperaturen von -10°C bis +30 °C.

Das erfindungsgemäße Verfahren (a) wird im Allgemeinen unter Normaldruck durchgeführt. Es ist jedoch auch möglich, unter erhöhtem Druck zu arbeiten, z. B., wenn das leicht flüchtige Difluoracetylfluorid oder -chlorid eingesetzt wird.

Die Reaktionszeit ist nicht kritisch und kann in Abhängigkeit von der Ansatzgröße in einem größeren Bereich gewählt werden.

Bei der Durchführung des erfmdungsgemäßen Verfahrens (a) setzt man auf 1 Mol Säurehalogenid der Formel (II) im Allgemeinen zwischen 0.5 Mol und 3 Mol, vorzugsweise zwischen 0.5 Mol und 1.5 Mol, besonders bevorzugt zwischen 0,9 Mol und 1,0 Mol eines Dialkylaminoacrylsäureesters der Formel (III) ein. Die Base wird im Allgemeinen äquimolar zum Säurehalogenid der Formel (II) eingesetzt. Vorzugsweise wird das im Lösungsmittel gelöste Säurehalogenid der Formel (II) vorgelegt und der Dialkylaminoacrylsäureester der Formel (III) zugegeben. Die umgekehrte Reihenfolge ist jedoch auch möglich. Anschließend wird die verwendete Base zudosiert.

Nach beendeter Reaktion kann das Hydrohalogenid des Pyridin-Derivats mit Wasser in Lösung gebracht werden bzw. die vorhandene wässrige Phase durch Phasentrennung entfernt werden. Die 2-Dihalogenacyl-3-amino-acrylsäureester der Formel (I) können in der verbleibenden organischen Phase ohne weitere Aufreinigung in die folgende Reaktionsstufe (Pyrazolsynthese) eingesetzt werden, gegebenenfalls nach Trocknen, z.B. durch Andestillieren des Lösungsmittels.

Die durch das erfindungsgemäßen Verfahren (a) herstellbaren 2-Dihalogenacyl-3-amino-acrylsäureester der Formel (I) sind neu und ebenfalls Gegenstand dieser Anmeldung.

2-Dihalogenacyl-3-amino-acrylsäureester der Formel (I) sind wertvolle Zwischenprodukte für die Herstellung von dihalogenmethyl-substituierten Pyrazolylcarbonsäure-Derivaten, welche wiederum Vorstufen von fungiziden Wirkstoffen darstellen (vgl. WO 03/070705).

Ebenfalls Gegenstand dieser Anmeldung ist daher die Verwendung von 2-Dihalogenacyl-3-amino-acrylsäureestern der Formel (I) zum Herstellen von 3-Dihalogenmethyl-1H-pyrazol-4-carbonsäureestern der Formel (V). Diese Ester können gegebenenfalls zur Säure verseift und optional weiter zu den entsprechenden Säurehalogeniden derivatisiert werden. Säure und Säurehalogenid können dann zu fungizid wirksamen Carboxamiden umgesetzt werden (vgl. WO 03/070705).

Ebenfalls Gegenstand der vorliegenden Erfindung ist ein Verfahren zum Herstellen von 3-Dihalogenmethyl-pyrazol-4-carbonsäureestern der Formel (V) 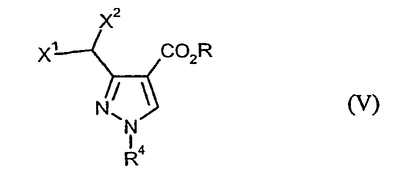 in welcher
- R: für C₁-C₄-Alkyl steht,
- X¹ und X²: unabhängig voneinander für Fluor, Chlor oder Brom stehen,
- R⁴: für C₁-C₄-Alkyl, Hydroxy-C₁-C₄-alkyl, C₂-C₆-Alkenyl, C₃-C₆-Cycloalkyl, C₁-C₄-Alkylthio-C₁- C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht,
**dadurch gekennzeichnet, dass** man
b) 2-Dihalogenacyl-3-amino-acrylsäureestern der Formel (I) 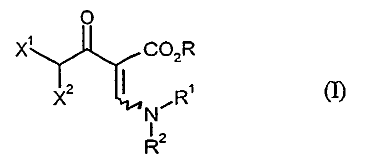 in welcher
   R, R¹ und R² unabhängig voneinander für C₁-C₄-Alkyl stehen und
   X¹ und X² unabhängig voneinander für Fluor, Chlor oder Brom stehen,
   mit Hydrazin-Derivaten der Formel (IV)

   R⁴-NH-NH₂ (IV)

   in welcher R⁴ die oben angegebenen Bedeutungen hat,
   bei Temperaturen von -50°C bis 0°C in Gegenwart eines aprotischen Lösungsmittels umsetzt.

Nach dem Stand der Technik erfolgt die Umsetzung von 2-Dihalogenacyl-3-amino-acrylsäureestern der Formel (I) mit Hydrazin-Derivaten üblicherweise in Ethanol als Lösungsmittel (vgl. US 5,498,624). Hierbei wird in beträchtlichem Umfang auch das falsche Pyrazol-Isomer (1-Methyl-5-difluormethyl-pyrazol-4-carbonsäure) erhalten (Verhältnis richtiges/falsches Isomer 65:35), welches durch Destillation entfernt werden kann. Die Ausbeute des gewünschten Isomeren beträgt nur 46.8 % der Theorie. Durch Anwendung der Bedingungen der WO 03/051820 auf die erfindungsgemäßen Dihalogenmethyl-Derivate wird ebenfalls nur ein Isomerenverhältnis von ca. 68:32 erhalten (Vergleichsbeispiel 3).

Überraschenderweise wurde nun gefunden, dass bei der Umsetzung von 2-Dihalogenacyl-3-amino-acrylsäureestern der Formel (I) mit Hydrazin-Derivaten der Formel (IV) (insbesondere mit Methylhydrazin) durch die Wahl der Temperatur, der Dosierung und des Lösungsmittels das Isomerenverhältnis von gewünschten zu unerwünschtem Pyrazol gesteuert und minimiert werden kann.

Verwendet man 2-(Dichloracetyl)-3-(dimethylamino)acrylsäureethylester und Methylhydrazin als Ausgangsstoffe, so kann der Verlauf des erfindungsgemäßen Verfahrens (b) durch das folgende Formelschema veranschaulicht werden.

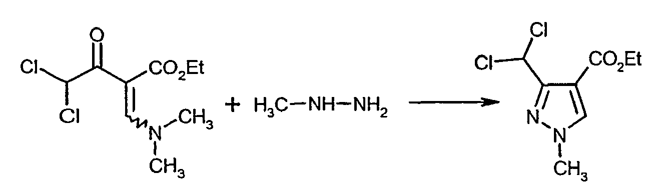

Die bei der Durchführung des erfindungsgemäßen Verfahrens (b) als Ausgangsstoffe benötigten 2-Dihalogenacyl-3-amino-acrylsäureestern der Formel (I) sind ebenfalls Teil dieser Erfindung und können gemäß dem erfindungsgemäßen Verfahren (a) hergestellt werden (vgl. oben).

Die bei der Durchführung des erfindungsgemäßen Verfahrens (b) als Ausgangsstoffe weiterhin benötigten Hydrazin-Derivate sind durch die Formel (IV) allgemein definiert. In dieser Formel steht R⁴ bevorzugt für Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl, besonders bevorzugt für Methyl, Ethyl, iso-Propyl, n-Propyl oder tert.-Butyl, ganz besonders bevorzugt für Methyl.

Die Hydrazin-Derivate der Formel (IV) sind bekannte Synthesechemikalien.

Nach dem erfindungsgemäßen Verfahren (b) wird der Ringschluss zum Pyrazol in Anwesenheit eines aprotischen Lösungsmittels durchgeführt. Hierzu gehören vorzugsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt verwendet man Toluol, Xylol, Chlorbenzol, n-Hexan, Cyclohexan oder Methylcyclohexan, ganz besonders bevorzugt Toluol oder Xylol.

Nach dem erfindungsgemäßen Verfahren (b) muss beim Ringschluss zum Pyrazol innerhalb eines relativ kleinen Temperaturbereichs gearbeitet werden. Je tiefer die Temperatur ist, um so vorteilhafter. Zu tiefe Temperaturen sind jedoch aus wirtschaftlicher Sicht nicht ökonomisch. Im Allgemeinen arbeitet man bei Temperaturen von -50°C bis +20°C, bevorzugt bei Temperaturen von -30°C bis 0°C, besonders bevorzugt von 20°C bis 0°C.

Bei dem erfindungsgemäßen Verfahren (b) wird der Ringschluss zum Pyrazol im Allgemeinen unter Normaldruck durchgeführt. Es ist jedoch auch möglich, unter erhöhtem oder vermindertem Druck zu arbeiten.

Nach dem erfindungsgemäßen Verfahren (b) kann beim Ringschluss zum Pyrazol entweder der 2-Dihalogenacyl-3-amino-acrylsäureester der Formel (I) oder das Hydrazin-Derivat vorgelegt werden. Um das gewünschte Pyrazol-Derivat in hoher Ausbeute und Selektivität zu erhalten, ist es jedoch entgegen dem Stand der Technik von Vorteil, das Hydrazin-Derivat vorzulegen und den Ester der Formel (I) dazu zu dosieren.

Nach beendeter Reaktion wird zunächst mit Wasser extrahiert, die organische Phase abgetrennt und das Lösungsmittel durch Destillation entfernt . Das falsche Isomer (1-Methyl-5-difluormethyl-pyrazol-4-carbonsäure) kann in manchen Fällen durch Kristallisation abgetrennt werden. Es ist auch möglich, zuerst zur Säure zu verseifen und anschließend umzukristallisieren.

Gegenstand dieser Erfindung ist außerdem das Gesamtverfahren zum Herstellen von 3-Dihalogenmethyl-1H-pyrazol-4-carbonsäureester der Formel (V), **dadurch gekennzeichnet, dass** man zunächst 2-Dihalogenacyl-3-amino-acrylsäureestern der Formel (I) gemäß dem erfindungsgemäßen Verfahren (a) herstellt und diese entweder nach Isolierung oder direkt weiter umsetzt und mit Hydrazin-Derivaten der Formel (IV) gemäß dem erfindungsgemäßen Verfahren (b) cyclisiert.

Einige der nach dem erfindungsgemäßen Verfahren (b) erhältlichen 3-Dihalogenmethyl-1H-pyrazol-4-carbonsäureester sind neu. Ebenfalls Gegenstand dieser Anmeldung sind 3-Dihalogenmethyl-1H-pyrazol-4-carbonsäureester der Formel (V-a) 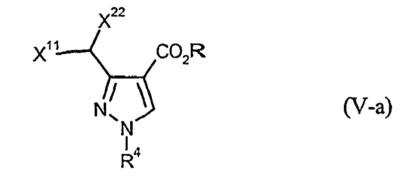 in welcher
- R: für C₁-C₄-Alkyl steht,
- X¹¹ und X²²: für Chlor stehen,
- R⁴: für C₁-C₄-Alkyl, Hydroxy-C₁-C₄-alkyl, C₂-C₆-Alkenyl, C₃-C₆-Cycloalkyl, C₁-C₄-Alkylthio-C₁- C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht.

Das erfindungsgemäße Herstellen von 2-Dihalogenacyl-3-amino-acrylsäureestern der Formel (I), sowie deren Verwendung zum Herstellen von dihalogenmethyl-substituierten Pyrazol-Derivaten der Formel (V) wird in den nachstehenden Beispielen beschrieben, welche die obige Beschreibung weiter illustrieren. Die Beispiele sind jedoch nicht in einschränkender Weise zu interpretieren.

### Herstellungsbeispiele

### Beispiel 1

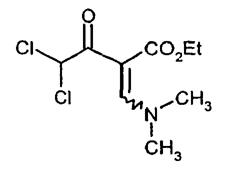

71.6 g (0.5 mol) Dimethylaminoacrylsäureethylester werden in 150 ml Toluol gelöst und die Mischung auf 0°C gekühlt. Anschließend tropft man innerhalb von 30-40 min bei 0-3°C 73.7 g (0.5 mol) Dichloracetylchlorid (gelöst in 50 ml Toluol) unter Rühren zu der Lösung. Dann werden bei der gleichen Temperatur innerhalb von 20 min 200 g 10%ige Natronlauge zugetropft, wobei sich eine gelbe Emulsion bildet. Man rührt 3 h bei 0-3°C nach und lässt auf Raumtemperatur kommen. Die Phasen werden getrennt. Die wässrige Phase wird mit 100 ml Toluol extrahiert und die vereinigten organischen Phasen mit 100 ml Wasser gewaschen. Die Toluolphase wird durch Andestillieren getrocknet und in die nächste Stufe eingesetzt (siehe Beispiel 4).

Das Produkt kann durch Abdestillieren des Toluols isoliert werden. Man erhält 101.9 g (80.2 % der Theorie) an 2-(Dichloracetyl)-3-(dimethylamino)-acrylsäureethylester.
¹H-NMR (CD₃CN): δ = 7.93 (s, 1H), 7.22 (s, 1H), 4.15-4.19 (m, 2H) 3.32(s, 3H), 2.85 (s, 3H), 1.25-1.29 (t, 3H) ppm.

### Beispiel 2

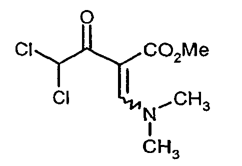

Man setzt 57.6 g Dimethylaminoacrylsäuremethylester analog Beispiel 1 um und erhält 95.2 g (79.3 % der Theorie) an 2-(Dichloracetyl)-3-(dimethylamino)-acrylsäuremethylester.

### Beispiel 3

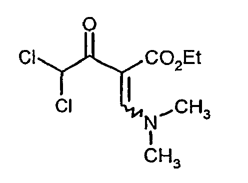

71.6 g (0.5 mol) Dimethylaminoacrylsäureethylester werden in 150 ml Toluol gelöst und unter Rühren bei 0-3°C zu einer Lösung von 73.7 g (0.5 mol) Dichloracetylchlorid in 50 ml Toluol getropft. Dann werden bei der gleichen Temperatur innerhalb von 20 min 200 g 10%ige Natronlauge zugetropft, wobei sich eine gelbe Emulsion bildet. Man rührt 3 h bei 0-3°C nach und lässt auf Raumtemperatur kommen. Die Phasen werden getrennt. Die wässrige Phase wird mit 100 ml Toluol extrahiert und die vereinigten organischen Phasen mit 100 ml Wasser gewaschen. Die Toluolphase wird durch Andestillieren getrocknet und in die nächste Stufe eingesetzt (siehe Beispiel 4).

Das Produkt kann durch Abdestillieren des Toluols isoliert werden. Man erhält 106.7 g (84 % der Theorie) an 2-(Dichloracetyl)-3-(dimethylamino)-acrylsäureethylester.

### Beispiel 4

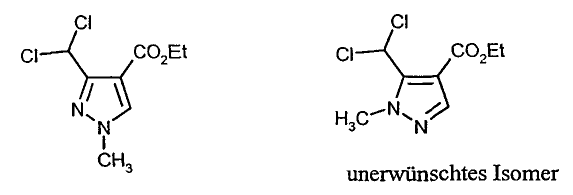

Zu 44.26 g Methylhydrazin in 940 ml Toluol werden unter Rühren und Schutzgas bei 0°C innerhalb von 45 min eine Lösung von 239.3 g 2-(Dichloracetyl)-3-(dimethylamino)-acrylsäureethylester in 940 ml Toluol getropft. Man rührt 3 h bei 0°C, lässt auf Raumtemperatur kommen und rührt noch 2 h bei 20-25°C. Nach Zugabe von 200 ml Wasser wird die Toluolphase abgetrennt und die Wasserphase noch zweimal mit je 200 ml Toluol extrahiert. Die vereinigten Toluolphasen werden mit 300 ml Wasser gewaschen und andestilliert.

Man erhält 3-(Dichlormethyl)-1-methyl-1H-pyrazol-4-carbonsäureethylester (Ausbeute 77.5 % der Theorie) im Gemisch mit dem unerwünschten Isomeren [5-(Dichlormethyl)-1-methyl-1H-pyrazol-4-carbonsäureethylester] im Verhältnis von 81.4 : 18.6 (GC/MS-Analytik). Der reine 3-(Dichlormethyl)-1-methyl-1H-pyrazol-4-carbonsäureethylester schmilzt bei 41°C.

Analog Beispiel 4 werden die Beispiele 5 bis 8 durchgeführt. Dabei werden jeweils das Lösungsmittel, die Reaktionstemperatur sowie die Reihenfolge der zugegebenen Reaktanden variiert. Je nach Wahl der Reaktionsbedingungen werden unterschiedliche Isomerenverhältnisse erhalten. Die Ergebnisse dieser Beispiele sind in der folgenden Tabelle 1 zusammengefasst.

**Tabelle 1**

| **Bsp.** | **Reaktionstemperatur** | **Lösungsmittel** | **Vorlage** | **Isomerenverhältnis** |
|---|---|---|---|---|
| 5 | -20°C | Toluol | Methylhydrazin | 87.3:12.7 |
| 6 | +20°C | Toluol | Methylhydrazin | 69.9:30.1 |
| 7 | +40°C | Toluol | Methylhydrazin | 61.7:38.3 |
| 8 | 0°C | Toluol | 2-(Dichloracetyl)-3-(dimethylamino)acrylsäureethylester | 67.9:32.1 |

### Beispiel 9

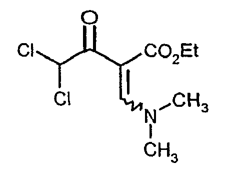

Zu 14.3 g (0.1 mol) Dimethylaminoacrylsäureethylester und 9.5 g (0.1 mol) 98%igen 2-Methylpyridin in 75 ml Toluol wird bei 0°C unter Rühren eine Lösung aus 14.7 g (0.1 mol) Dichloracetylchlorid in 25 ml Toluol getropft. Man lässt auf Raumtemperatur kommen und rührt 30 min nach. Nach Zugabe von 100 ml Wasser werden die Phasen getrennt, die wässrige Phase mit 50 ml Toluol extrahiert und die vereinigten organischen Phasen mit 50 ml Wasser gewaschen. Die Toluolphase wird durch Andestillieren getrocknet und in die nächste Stufe eingesetzt (vgl. Beispiel 4).

Durch Abdestillieren des Toluols kann das Produkt isoliert werden. Man erhält 23.2 g (91.3 % der Theorie) an 2-(Dichloracetyl)-3-(dimethylamino)-acrylsäureethylester (Fp. 71-72°C).

### Beispiel 10

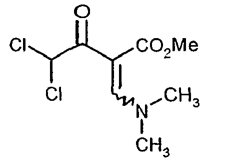

Zu 6.9 g (0.05 mol) 93%igen Dimethylaminoacrylsäuremethylester und 4.7 g (0.05 mol) 98%igen 2-Methylpyridin gelöst in 40 ml Toluol wird bei 0°C unter Rühren eine Lösung aus 7.4 g (0.05 mol) Dichloracetylchlorid in 15 ml Toluol getropft. Man lässt auf Raumtemperatur kommen und rührt 30 Minuten nach. Nach Zugabe von 50 ml Wasser werden die Phasen getrennt, die wässrige Phase mit 50 ml Toluol extrahiert und die vereinigten organischen Phasen mit 50 ml Wasser gewaschen. Die Toluolphase wird durch Andestillieren getrocknet und in die nächste Stufe eingesetzt (vgl. Beispiel 11). Durch Abdestillieren des Toluols kann das Produkt isoliert werden. Man erhält 10.4 g (95%ig, 82.3 % der Theorie) an 2-(Dichloracetyl)-3-(dimethylamino)-acrylsäuremethylester.

### Beispiel 11

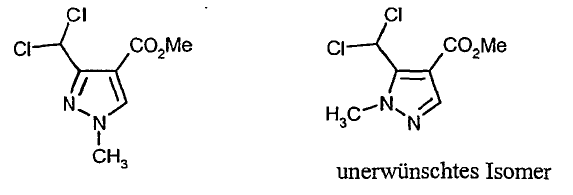

Zu 2.8 g (0.06 mol) Methylhydrazin in 60 ml Toluol wird unter Schutzgas und Rühren bei -50°C innerhalb von 45 min eine Lösung aus 15.2 g (0.06 mol) 2-(Dichloracetyl)-3-(dimethylamino)-acrylsäuremethylester in 60 ml Toluol getropft. Man rührt 30 min bei -50°C, lässt auf Raumtemperatur kommen und rührt noch 2 h nach. Nach Zugabe von 60 ml Wasser werden die Phasen getrennt, die wässrige Phase mit 30 ml Toluol extrahiert, die organischen Phasen vereinigt und mit 30 ml Wasser gewaschen.

Nach Abdestillieren des Toluols erhält man 12.6 g (83 % der Theorie) an 3-(Dichlormethyl)-1-methyl-1H-pyrazol-4-carbonsäuremethylester im Gemisch mit dem unerwünschten Isomeren [5-(Dichlormethyl)-1-methyl-1H-pyrazol-4-carbonsäuremethylester] im Verhältnis 93.8:6.2 (GC/MS-Analytik).

Das Produkt kann aus Diisopropylether umkristallisiert werden (Fp. 115°C).

### Beispiel 12

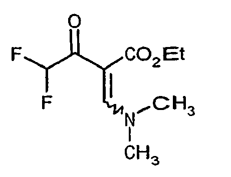

Zu 26 g Difluoracetylchlorid in 200 ml Toluol wird unter Rühren und Schutzgas bei 0°C 32.5 g Dimethylaminoacrylsäureethylester in 200 ml Toluol zugetropft. Anschließend tropft man 90.85 g 10%ige Natronlauge zu. Man rührt 3 h bei 0°C nach und lässt auf Raumtemperatur kommen. Die Phasen werden getrennt. Die wässrige Phase wird mit 80 ml Toluol extrahiert und die vereinigten organischen Phasen mit 80 ml Wasser gewaschen. Die Toluolphase wird durch Andestillieren getrocknet und in die nächste Stufe eingesetzt (siehe Beispiel 11).

Das Produkt kann durch Abdestillieren des Toluols isoliert werden. Man erhält 37.2 g (74 % der Theorie) an 2-(Difluoracetyl)-3-(dimethylamino)-acrylsäureethylester.
¹H-NMR (CD₃CN): δ = 7.88 (s, 1H), 6,47, 6,61 und 6.74 (t, 1H), 4.13-4.19 (m, 2H), 3.32 (s, 3H), 2.85 (s, 3H), 1.25-1.28 (t, 3H) ppm.

### Beispiel 13

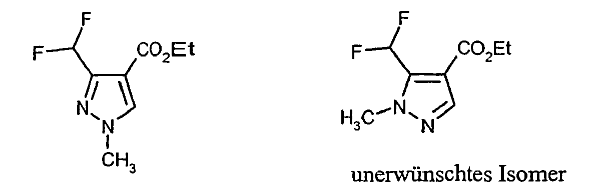

Zu 31.2 g Methylhydrazin in 600 ml Toluol werden unter Rühren und Schutzgas bei -20°C innerhalb von 45 min eine Lösung von 125.3 g 2-(Difluoracetyl)-3-(dimethylamino)-acrylsäureethylester in 400 ml Toluol getropft. Man rührt 3 h bei 0°C, lässt auf Raumtemperatur kommen und rührt noch 2 h bei 20-25°C. Nach Zugabe von 500 ml Wasser wird die Toluolphase abgetrennt und die Wasserphase noch zweimal mit je 100 ml Toluol extrahiert. Die vereinigten Toluolphasen werden mit 300 ml Wasser gewaschen und andestilliert.

Man erhält 3-(Difluormethyl)-1-methyl-1H-pyrazol-4-carbonsäureethylester (Ausbeute 89.7 % der Theorie) im Gemisch mit dem unerwünschten Isomeren [5-(Difluormethyl)-1-methyl-1H-pyrazol-4-carbonsäureethylester] im Verhältnis von 89.2 : 10.8 (GC/MS-Analytik).

### Beispiel 14

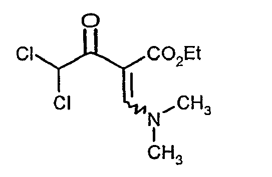

Zu einer Lösung aus 28.9 g (0.2 mol) Dimethylaminoacrylsäureethylester und 24.7 g (0.2 mol) 5-Ethyl-2-methylpyridin in 150 ml Toluol wird innerhalb von 15 Minuten bei Raumtemperatur 30.4 g (0.2 mol) Dichloracetylchlorid in 50 ml Toluol getropft. Die entstandene gelbe Suspension wird 2 Stunden bei gleicher Temperatur gerührt. Nach Zugabe von 200 ml Wasser werden die Phasen getrennt, die wässrige Phase mit 50 ml Toluol extrahiert und die vereinigten organischen Phasen mit 50 ml Wasser gewaschen, getrocknet, eingeengt und im Hochvakuum "entgast".

Man erhält 47.6 g (92.1 % der Theorie) an 2-(Dichloracetyl)-3-(dimethylamino)-acrylsäureethylester (Fp. 73°C).

### Vergleichsbeispiel 1 (analog Beispiel aus US 5,498,624, Spalten 4 und 5)

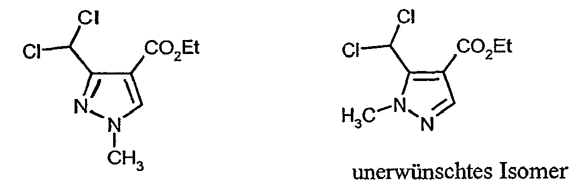

Zu 7.1 g (0.028 mol) 2-Dichloracetyl-3-(dimethylamino)-acrylsäureethylester in 35 ml Ethanol (absolut) wird unter Schutzgas und Rühren bei 0°C eine Lösung aus 1.3 g (0.028 mol) Methylhydrazin in 5 ml Ethanol (absolut) innerhalb von 40 min zugetropft. Man erhitzt 2 h auf Rückfluss, lässt auf Raumtemperatur kommen und rührt die Lösung noch 2 h nach. Nach Abdestillieren des Ethanols werden 50 ml Wasser und 50 ml Dichlormethan zugegeben, die Phasen getrennt, die wässrige Phase mit 30 ml Dichlormethan extrahiert, die organischen Phasen vereinigt und mit 30 ml Wasser gewaschen.

Nach Abdestillieren des Dichlormethans erhält man 6.5 g (37.5 % der Theorie) an 3-(Dichlormethyl)-1-methyl-1H-pyrazol-4-carbonsäureethylester im Gemisch mit dem unerwünschten Isomeren im Verhältnis 40.3 : 59.7.

### Vergleichsbeispiel 2 (analog Beispiel 3 aus WO 03/051820)

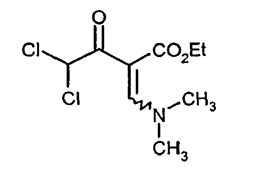

Zu 14.3 g (0.1 mol) Dimethylaminoacrylsäureethylester und 11.1 g (0.11 mol) Triethylamin in 75 ml Toluol, wird bei -10° bis -5°C unter Rühren eine Lösung aus 16.2 g (0.11 mol) Dichloracetylchlorid in 25 ml Toluol getropft und anschließend auf 50°C erwärmt. Nach Zugabe von 100 ml Wasser werden die Phasen getrennt, die wässrige Phase mit 50 ml Toluol extrahiert und die vereinigten organischen Phasen mit 50 ml Wasser gewaschen.

Nach Abdestillieren des Toluols erhält man 23.7 g eines dunklen Öls, welches zu 47.8 % 2-(Dichloracetyl)-3-(dimethylamino)-acrylsäureethylester enthält (entspricht einer theoretischen Ausbeute von 44.6 %).

### Vergleichsbeispiel 3 (analog Beispiel 4 aus WO 03/051820)

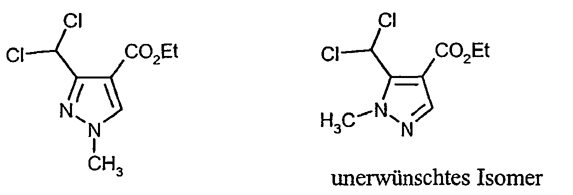

Zu 12.7 g (0.05 mol) 2-(Dichloracetyl)-3-(dimethylamino)-acrylsäureethylester in 50 ml Toluol wurde bei 0°C eine Lösung aus 2.3 g (0.05 mol) Methylhydrazin in 10 ml Toluol getropft. Anschließend ließ man die Mischung noch 1 h nachrühren. Das Toluol wurde unter vermindertem Druck (< 100 mbar) und Temperaturen von max. 45°C abdestilliert. Das Produkt kristallisierte mit Wasser nicht. Es wurde zweimal mit Dichlormethan extrahiert, die vereinigten, organischen Phasen mit Wasser gewaschen und eingeengt.

Man erhielt 11.1 g an 3-(Dichlormethyl)-1-methyl-1H-pyrazol-4-carbonsäureethylester als rotes Öl im Gemisch mit dem unerwünschten Isomeren [5-(Dichlormethyl)-1-methyl-1H-pyrazol-4-carbonsäureethylester] im Verhältnis von 67,9 : 32,1 (GC/MS-Analytik).

## Patentansprüche

1. Verfahren zum Herstellen von 2-Dihalogenacyl-3-amino-acrylsäureestern der Formel (I) 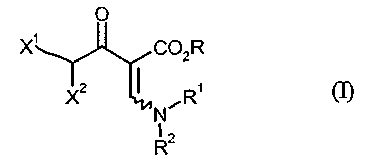 in welcher
R, R¹ und R² unabhängig voneinander für C₁-C₄-Alkyl stehen und
X¹ und X² unabhängig voneinander für Fluor, Chlor oder Brom stehen,
**dadurch gekennzeichnet, dass** man
Säurehalogenide der Formel (II) 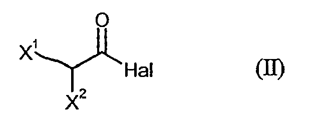 in welcher
Hal für Fluor, Chlor oder Brom steht und
X¹ und X² unabhängig voneinander für Fluor, Chlor oder Brom stehen,
mit Dialkylaminoacrylsäureestern der Formel (III) 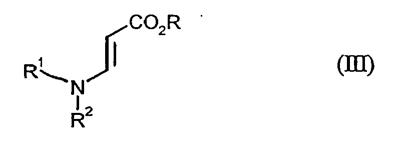 in welcher R, R¹ und R² die oben angegebenen Bedeutungen haben,
in einem mit Wasser nicht mischbaren organischen Lösungsmittel in Gegenwart einer Base umsetzt.

2. Verfahren nach Anspruch 1 **dadurch gekennzeichnet, dass** als Base Pyridin, Picolin, 2-Methyl-5-ethyl-pyridin, 2,4,6-Collidin, Chinolin oder Chinaldin verwendet wird.

3. 2-Dihalogenacyl-3-amino-acrylsäureester der Formel (I) 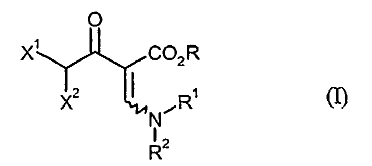 in welcher
R, R¹ und R² unabhängig voneinander für C₁-C₄-Alkyl stehen und
X¹ und X² unabhängig voneinander für Fluor, Chlor oder Brom stehen.

4. Verwendung von 2-Dihalogenacyl-3-amino-acrylsäureestern der Formel (I) zum Herstellen von 3-Dihalogenmethyl-1H-pyrazol-4-carbonsäureestern der Formel (V) 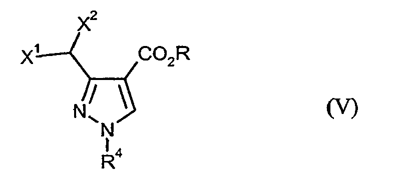 in welcher
R für C₁-C₄-Alkyl steht,
X¹ und X² unabhängig voneinander für Fluor, Chlor oder Brom stehen,
R⁴ für C₁-C₄-Alkyl, Hydroxy-C₁-C₄-alkyl, C₂-C₆-Alkenyl, C₃-C₆-Cycloalkyl, C₁-C₄- Alkylthio-C₁-C₄-alkyl, C₁-C₄-Alkoxy-C₁-C₄-alkyl, C₁-C₄-Halogenalkyl, C₁-C₄- Halogenalkylthio-C₁-C₄-alkyl, C₁-C₄-Halogenalkoxy-C₁-C₄-alkyl mit jeweils 1 bis 5 Halogenatomen, oder für Phenyl steht.

5. Verfahren zum Herstellen von 3-Dichlormethyl-pyrazol-4-carbonsäureestern der Formel (V) 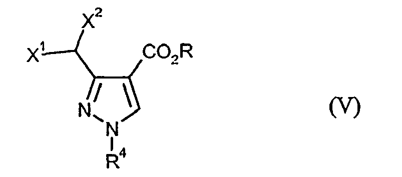 in welcher
R für C₁-C₄-Alkyl steht,
X¹ und X² unabhängig voneinander für Fluor, Chlor oder Brom stehen,
R⁴ für C₁-C₄-Alkyl, Hydroxy-C₁-C₄-alkyl, C₂-C₆-Alkenyl, C₃-C₆-Cycloalkyl, C₁-C₄- Alkylthio-C₁-C₄-alkyl, C₁-C₄-Alkoxy-C₁-C₄-alkyl, C₁-C₄-Halogenalkyl, C₁-C₄- Halogenalkylthio-C₁-C₄-alkyl, C₁-C₄-Halogenalkoxy-C₁-C₄-alkyl mit jeweils 1 bis 5 Halogenatomen, oder für Phenyl steht,
**dadurch gekennzeichnet, dass** man 2-Dihalogenacyl-3-amino-acrylsäureestern der Formel (I) 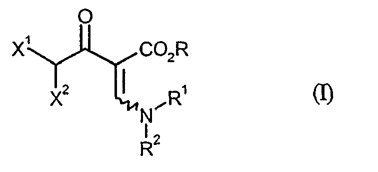 in welcher
R, R¹ und R² unabhängig voneinander für C₁-C₄-Alkyl stehen und
X¹ und X² unabhängig voneinander für Fluor, Chlor oder Brom stehen,
mit Hydrazin-Derivaten der Formel (IV)
R⁴-NH-NH₂ (IV)
in welcher R⁴ die oben angegebenen Bedeutungen hat,
bei Temperaturen von -50°C bis 0°C in Gegenwart eines aprotischen Lösungsmittels umsetzt.

6. Verfahren zum Herstellen von 3-Dichlormethyl-pyrazol-4-carbonsäureestern der Formel (V) gemäß Anspruch 5, **dadurch gekennzeichnet, dass** man die 2-Dihalogenacyl-3-aminoacrylsäureester der Formel (I) nach dem Verfahren gemäß Anspruch 1 herstellt.

7. 3-Dichlormethyl-pyrazol-4-carbonsäureester der Formel (V-a) 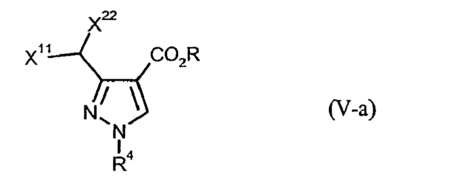 in welcher
R für C₁-C₄-Alkyl steht,
X¹¹ und X²² für Chlor stehen,
R⁴ für C₁-C₄-Alkyl, Hydroxy-C₁-C₄-alkyl, C₂-C₆-Alkenyl, C₃-C₆-Cycloalkyl, C₁-C₄- Alkylthio-C₁-C₄-alkyl, C₁-C₄-Alkoxy-C₁-C₄-alkyl, C₁-C₄-Halogenalkyl, C₁-C₄-Hal- genalkylthio-C₁-C₄-alkyl, C₁-C₄-Halogenalkoxy-C₁-C₄-alkyl mit jeweils 1 bis 5 Halo- genatomen, oder für Phenyl steht.

## Claims

1. Process for preparing 2-dihaloacyl-3-aminoacrylic esters of the formula (I) 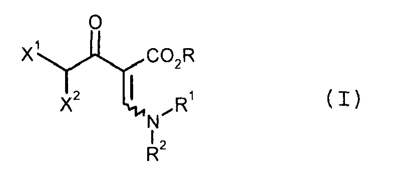 in which
R, R¹ and R² are each independently C₁-C₄-alkyl and
X¹ and X² are each independently fluorine, chlorine or bromine,
**characterized in that**
acid halides of the formula (II) 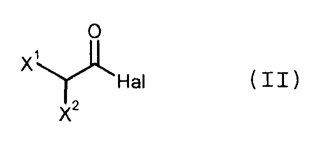 in which
Hal is fluorine, chlorine or bromine and
X¹ and X² are each independently fluorine, chlorine or bromine
are reacted with dialkylaminoacrylic esters of the formula (III) 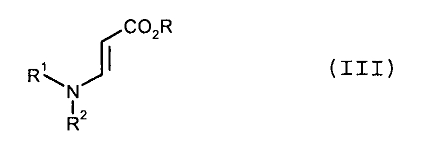 in which R, R¹ and R² are each as defined above
in a water-immiscible organic solvent in the presence of a base.

2. Process according to Claim 1, **characterized in that** the base used is pyridine, picoline, 2-methyl-5-ethylpyridine, 2,4,6-collidine, quinoline or quinaldine.

3. 2-Dihaloacyl-3-aminoacrylic esters of the formula (I) 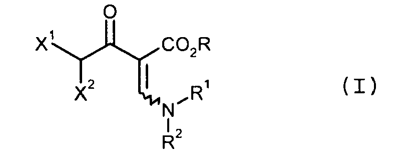 in which
R, R¹ and R² are each independently C₁-C₄-alkyl and X¹ and X² are each independently fluorine, chlorine or bromine.

4. Use of 2-dihaloacyl-3-aminoacrylic esters of the formula (I) to prepare 3-dihalomethyl-1H-pyrazole-4-carboxylic esters of the formula (V) 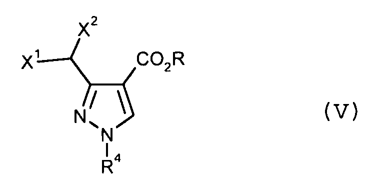 in which
R is C₁-C₄-alkyl,
X¹ and X² are each independently fluorine, chlorine or bromine,
R⁴ is C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄-
alkoxy-C₁-C₄-alkyl, C₁-C₄-haloalkyl, C₁-C₄- haloalkylthio-C₁-C₄-alkyl, C₁-C₄-haloalkoxy-C₁-C₄- alkyl having in each case 1 to 5 halogen atoms, or is phenyl.

5. Process for preparing 3-dichloromethylpyrazole-4-carboxylic esters of the formula (V) 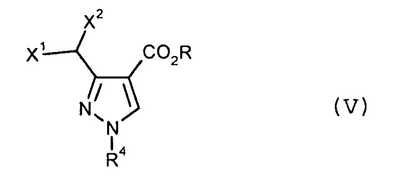 in which
R is C₁-C₄-alkyl,
X¹ and X² are each independently fluorine, chlorine or bromine,
R⁴ is C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄- alkoxy-C₁-C₄-alkyl, C₁-C₄-haloalkyl, C₁-C₄- haloalkylthio-C₁-C₄-alkyl, C₁-C₄-haloalkoxy-C₁-C₄- alkyl having in each case 1 to 5 halogen atoms, or is phenyl,
**characterized in that** 2-dihaloacyl-3-aminoacrylic esters of the formula (I) 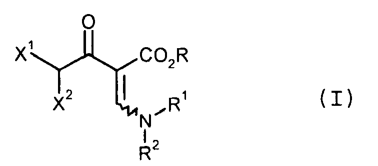 in which
R, R¹ and R² are each independently C₁-C₄-alkyl and
X¹ and X² are each independently fluorine, chlorine or
bromine
are reacted with hydrazine derivatives of the formula (IV)
R⁴-NH-NH₂ (IV)
in which R⁴ is as defined above
at temperatures of -50°C to 0°C in the presence of an aprotic solvent.

6. Process for preparing 3-dichloromethylpyrazole-4-carboxylic esters of the formula (V) according to Claim 5, **characterized in that** 2-dihaloacyl-3-aminoacrylic esters of the formula (I) are prepared by the process according to Claim 1.

7. 3-Dichloromethylpyrazole-4-carboxylic esters of the formula (V-a) 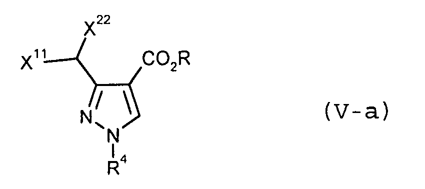 in which
R is C₁-C₄-alkyl,
X¹¹ and X²² are each chlorine,
R⁴ is C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄- alkoxy-C₁-C₄-alkyl, C₁-C₄-haloalkyl, C₁-C₄- haloalkylthio-C₁-C₄-alkyl, C₁-C₄-haloalkoxy-C₁-C₄- alkyl having in each case 1 to 5 halogen atoms, or is phenyl.

## Revendications

1. Procédé de fabrication d'esters d'acide 2-dihalogénoacyl-3-amino-acrylique de formule (I) 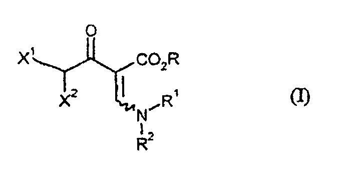 dans laquelle
R, R¹ et R² représentent indépendamment les uns des autres un alkyle en C₁-C₄ et
X¹ et X² représentent indépendamment l'un de l'autre le fluor, le chlore ou le brome,
**caractérisé en ce que**
des halogénures d'acide de formule (II) 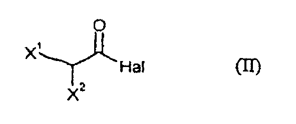 dans laquelle
Hal représente le fluor, le chlore ou le brome, et
X¹ et X² représentent indépendamment l'un de l'autre le fluor, le chlore ou le brome,
sont mis en réaction avec des esters d'acide dialkylaminoacrylique de formule (III) 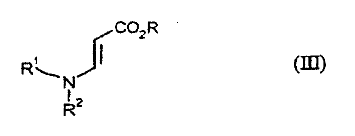 dans laquelle R, R¹ et R² ont les significations données ci-dessus,
dans un solvant organique non miscible avec l'eau en présence d'une base.

2. Procédé selon la revendication 1, **caractérisé en ce que** de la pyridine, de la picoline, de la 2-méthyl-5-éthyl-pyridine, de la 2,4,6-collidine, de la quinoline ou de la quinaldine est utilisée en tant que base.

3. Ester d'acide 2-dihalogénoacyl-3-amino-acrylique de formule (I) 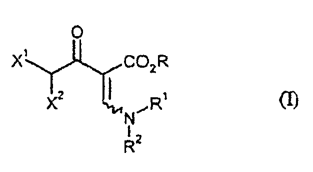 dans laquelle
R, R¹ et R² représentent indépendamment les uns des autres un alkyle en C₁-C₄ et
X¹ et X² représentent indépendamment l'un de l'autre le fluor, le chlore ou le brome.

4. Utilisation d'esters d'acide 2-dihalogénoacyl-3-amino-acrylique de formule (I) pour la fabrication d'esters d'acide 3-dihalogénométhyl-1H-pyrazol-4-carboxylique de formule (V) 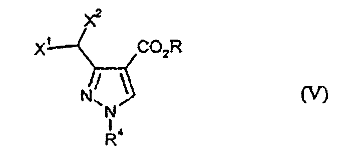 dans laquelle
R représente un alkyle en C₁-C₄,
X¹ et X² représentent indépendamment l'un de l'autre le fluor, le chlore ou le brome,
R⁴ représente un alkyle en C₁-C₄, un hydroxyalkyle en C₁-C₄, un alcényle en C₂-C₆, un cycloalkyle en C₃-C₆, un alkylthio en C₁-C₄-alkyle en C₁-C₄, un alcoxy en C₁-C₄-alkyle en C₁-C₄, un halogénoalkyle en C₁-C₄, un halogénoalkyle en C₁-C₄-thioalkyle en C₁-C₄, un halogénoalcoxy en C₁-C₄-alkyle en C₁-C₄ contenant à chaque fois 1 à 5 atomes d'halogène, ou un phényle.

5. Procédé de fabrication d'esters d'acide 3-dichlorométhyl-pyrazol-4-carboxylique de formule (V) 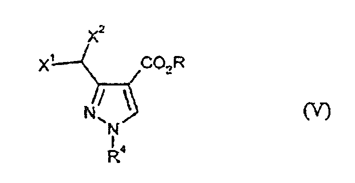 dans laquelle
R représente un alkyle en C₁-C₄,
X¹ et X² représentent indépendamment l'un de l'autre le fluor, le chlore ou le brome,
R⁴ représente un alkyle en C₁-C₄, un hydroxyalkyle en C₁-C₄, un alcényle en C₂-C₆, un cycloalkyle en C₃-C₆, un alkylthio en C₁-C₄-alkyle en C₁-C₄, un alcoxy en C₁-C₄-alkyle en C₁-C₄, un halogénoalkyle en C₁-C₄, un halogénoalkyle en C₁-C₄-thioalkyle en C₁-C₄, un halogénoalcoxy en C₁-C₄-alkyle en C₁-C₄ contenant à chaque fois 1 à 5 atomes d'halogène, ou un phényle,
**caractérisé en ce que** des esters d'acide 2-dihalogénoacyl-3-amino-acrylique de formule (I) 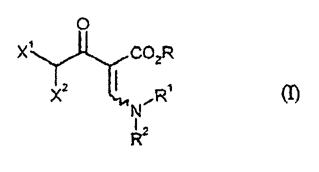 dans laquelle
R, R¹ et R² représentent indépendamment les uns des autres un alkyle en C₁-C₄ et
X¹ et X² représentent indépendamment l'un de l'autre le fluor, le chlore ou le brome,
sont mis en réaction avec des dérivés d'hydrazine de formule (IV)
R⁴-NH-NH₂ (IV)
dans laquelle R⁴ a les significations données ci-dessus, à des températures de -50 °C à 0 °C en présence d'un solvant aprotique.

6. Procédé de fabrication d'esters d'acide 3-dichlorométhyl-pyrazol-4-carboxylique de formule (V) selon la revendication 5, **caractérisé en ce que** l'ester d'acide 2-dihalogénoacyl-3-amino-acrylique de formule (I) est fabriqué par le procédé selon la revendication 1.

7. Ester d'acide 3-dichlorométhyl-pyrazol-4-carboxylique de formule (V-a) 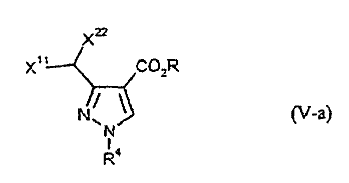 dans laquelle
R représente un alkyle en C₁-C₄,
X¹¹ et X²² représentent le chlore,
R⁴ représente un alkyle en C₁-C₄, un hydroxyalkyle en C₁-C₄, un alcényle en C₂-C₆, un cycloalkyle en C₃-C₆, un alkylthio en C₁-C₄-alkyle en C₁-C₄, un alcoxy en C₁-C₄-alkyle en C₁-C₄, un halogénoalkyle en C₁-C₄, un halogénoalkyle en C₁-C₄-thioalkyle en C₁-C₄, un halogénoalcoxy en C₁-C₄-alkyle en C₁-C₄ contenant à chaque fois 1 à 5 atomes d'halogène, ou un phényle.
